# FASCICULE DE BREVET EUROPEEN

(11) **EP 1 265 614 B1**
(45) Date de publication et mention de la délivrance du brevet: **01.06.2005**
(21) Numéro de dépôt: 01911692.0
(22) Date de dépôt: 22.02.2001
(51) Int. Cl.: A61K 31/535, A61K 9/20

(54) **NOUVELLE FORME GALENIQUE ORALE A LIBERATION PROLONGEE DE LA MOLSIDOMINE**
MOLSIDOMINHALTIGE ORAL ANZUWENDENDE VERABREICHUNGSFORM MIT VERZÖGERTER WIRKSTOFFABGABE
NOVEL GALENICAL FORM FOR ORAL ADMINISTRATION WITH PROLONGED RELEASE OF MOLSIDOMINE

(30) Priorité: 24.02.2000 FR 0002307
(43) Date de publication de la demande: 18.12.2002
(73) Titulaire: Therabel Pharmaceuticals Limited, Loughrea, C. Galway (IE)
(72) Inventeur: GECZY, Jozsef-Michel, B-1180 Bruxelles (BE)
(74) Mandataire: Hubert, Philippe
(86) Numéro de dépôt international: PCT/EP2001/002055
(87) Numéro de publication internationale: WO 2001/062256

(56) Documents cités:
- EP-A- 0 624 370
- EP-A- 0 714 661
- WO-A-91/14680

## Description

La présente invention concerne une nouvelle forme galénique orale à libération prolongée de la molsidomine destinée au traitement de l'angine de poitrine sous toutes ses formes (angor d'effort ou de repos, angor instable).

On sait que la molsidomine (ou N-(éthoxycarbonyl)3-(4-morpholinyl) sydnonimine) est le premier représentant d'une nouvelle famille d'anti-angineux. les sydnonimines, lequel a été décrit dans le brevet spécial de médicament No. 6734.

Ce composé est particulièrement utile pour le traitement préventif de la crise angineuse sous toutes ses formes, dans la mesure où il agit en provoquant une relaxation de la fibre musculaire lisse vasculaire, et une inhibition des phases précoces de l'activation plaquettaire.

L'activité de ce composé est attribuée à sa faculté de libérer directement le radical NO au cours de sa biotransformation.

Plus précisément la molsidomine est une prodrogue.

Après prise orale, la molsidomine est complètement absorbée et elle subit une transformation (hydrolyse et décarboxylation) enzymatique au niveau hépatique. Le SIN-1 ainsi produit est lui-même transformé rapidement au niveau sanguin et sans intervention enzymatique en SIN-1A. Le SIN-1 et le SIN-1A sont les métabolites actifs de la molsidomine.

Le SIN-1A par oxydation est ensuite dégradé en SIN-1C inactif avec libération du NO.

Le SIN-1C est ensuite lui-même métabolisé dans le foie comme décrit dans le document Bemd Rosenkranz and al., Clinical Pharmacokinetics of Molsidomine, Clin. Pharmacokinet. 1996, May ; 30 (5) 372-384.

La molsidomine est actuellement commercialisée essentiellement sous forme de comprimés sécables dosés à 2 mg et 4 mg, généralement administrés trois fois par jour dans le traitement de l'angor d'effort, et quatre fois par jour dans le traitement de l'angor de repos et de l'angor d'effort sévère.

Plus récemment, il a été proposé une nouvelle forme galénique orale à libération prolongée de la molsidomine dosée à 8 mg et destinée à être administrée deux fois par jour pour un traitement prophylactique et au long cours de l'angine de poitrine.

Sous cette forme, la concentration plasmatique maximale de la molsidomine est observée entre 1 et 3 heures après administration.

La molsidomine agit généralement pendant une période de 4 à 5 heures lorsqu'elle est dosée à 4 mg, et pendant une période de 10 à 12 heures, lorsqu'elle est dosée à 8 mg.

D'une façon générale, il est avantageux, du point de vue du confort du patient, de disposer de formes galéniques présentant un effet thérapeutique plus long permettant de réduire, par voie de conséquence, le nombre de prises quotidiennes du médicament, la réduction du nombre de prises assurant une meilleure compliance du patient.

Cependant, il est connu dans l'art pharmaceutique que l'allongement de l'effet thérapeutique implique une diminution significative de la concentration plasmatique maximale, et une entrée retardée dans la zone thérapeutique.

Il a été découvert, et ceci constitue le fondement de la présente invention, qu'en ce qui concerne la molsidomine et ses métabolites actifs, l'allongement de la couverture thérapeutique peut être obtenu, sans diminution significative de la concentration plasmatique maximale, avec une entrée dans la zone thérapeutique comparable à celle obtenue avec des formes dosées à 4 mg, ou 8 mg.

Ainsi la nouvelle forme galénique conforme à l'invention, bien qu'ayant des propriétés de libération prolongée, libère une proportion suffisante et calibrée de son principe actif en milieu acide et donc principalement dans l'estomac, ce qui assure une entrée rapide (environ 30 min à jeun à 1 h 30 en situation postprandiale) dans la zone thérapeutique (5 à 10 ng/ml) et un pic plasmatique équivalent (33 à 40 ng/ml) à celui mesuré avec les formes galéniques à libération immédiate.

L'importance du passage stomacal ou en d'autres termes, du milieu acide, a pu être démontrée grâce à des mesures de corrélations in vivo - in vitro. C'est en milieu HCl 0.1 N que la corrélation entre le pourcentage de libération in vitro et le pourcentage d'absorption in vivo est la plus élevée et ce, pour toutes les formes de molsidomine.

Ainsi, selon un premier aspect, la présente invention a pour objet une forme galénique orale solide à libération prolongée de la molsidomine. caractérisée en ce qu'elle contient une quantité thérapeutiquement efficace de molsidomine ou de l'un de ses métabolites actifs et en ce qu'elle présente un taux de dissolution in vitro [mesuré spectrophotométriquement à 286 ou 311 nm selon la méthode décrite dans la Pharmacopée Européenne, 3ème édition (ou U.S.P. XXIV) à 50 t.p.m. dans 500 ml d'un milieu HCl 0,1 N, à 37°C] de :
- 15 à 25 % de molsidomine libérée après 1 heure
- 20 à 35 % de molsidomine libérée après 2 heures
- 50 à 65 % de molsidomine libérée après 6 heures
- 75 à 95 % de molsidomine libérée après 12 heures
- > 85 % de molsidomine libérée après 18 heures
- > 90 % de molsidomine libérée après 24 heures,
le pic plasmatique de molsidomine obtenu in vivo se présentant dans les 2,5 à 5 heures, de préférence dans les 3 à 4 heures, suivant l'administration de ladite forme et ayant une valeur comprise entre 25 et 40 ng/ml de plasma.

Dans la présente description, '' le pic plasmatique de molsidomine obtenu in vivo " correspond à la concentration maximale moyenne de molsidomine trouvée dans le plasma d'au moins 10 volontaires en bonne santé.

L'expression " quantité thérapeutiquement efficace " utilisée dans le cadre de la présente invention signifie une quantité de molsidomine suffisante pour fournir une concentration plasmatique d'au moins 5 ng/ml, et de préférence d'au moins 10 ng/ml de plasma, pendant une période d'environ 24 heures.

D'une façon générale, la forme galénique conforme à la présente invention peut contenir de 14 à 24 mg, et de préférence de 16 à 20 mg de molsidomine par unité de dosage, la forme actuellement préférée contenant 16 mg de molsidomine.

Par l'expression " métabolites actifs " de la molsidomine on vise à couvrir notamment les composés SIN-1 et SIN-1A résultant de la biotransformation subie par la molsidomine après son administration.

La nouvelle forme galénique conforme à l'invention présente de nombreux avantages par rapport aux formes galéniques de la molsidomine actuellement commercialisées.

Tout d'abord, elle offre un grand confort au patient, puisqu'une prise quotidienne unique est suffisante pour obtenir l'effet thérapeutique recherché. La compliance des patients s'en trouve accrue.

Ensuite, le maintien d'une concentration plasmatique maximale élevée garantit une efficacité optimale pendant les premières heures après l'administration avec une entrée dans la zone thérapeutique (5 à 10 ng/ml) très rapide (30 minutes à jeun et 1 h 30 après repas).

De ce fait, cette nouvelle forme galénique évite :
- d'une part, les éventuelles périodes pendant lesquelles le patient ne serait pas protégé (vallées avec une concentration inférieure à 5 - 10 ng/ml) ; et
- d'autre part, les effets secondaires générés par l'induction de plusieurs pics plasmatiques journaliers corrélatifs à l'administration journalière en plusieurs prises.

Par ailleurs, une étude clinique préliminaire a montré, d'une façon tout à fait inattendue, que des patients dont l'état est stabilisé par un traitement bi-journalier par la molsidomine (comprimés à libération prolongée dosés à 8 mg) accompagné de la prise de dérivés nitrés organiques par voie sublinguale lors des crises présentent, après traitement subséquent par une forme galénique conforme à l'invention (dosée par exemple à 16 mg), une diminution significative des crises angineuses, et par voie de conséquence, de la consommation de dérivés nitrés organiques.

La nouvelle forme galénique conforme à la présente invention peut se présenter par exemple, sous forme de comprimés, sous forme pluriparticulaire ou sous forme de sphéroïdes, la forme comprimée étant préférée.

Avantageusement, la molsidomine est incorporée dans un système de libération, permettant l'obtention des taux de dissolution in vitro particuliers définis précédemment.

Ce système de libération peut être constitué par exemple d'une matrice à libération prolongée, ou bien encore d'une formulation traditionnelle comprenant un enrobage permettant la libération prolongée de la molsidomine.

Selon une caractéristique particulière de la présente invention, ce système de libération est constitué d'une matrice active comprenant, mélangées à la molsidomine ou à l'un de ses métabolites actifs, une matière polymère avant un pouvoir de gonflement élevé au contact de l'eau ou de liquides aqueux, une matière polymère gélifiable, lesdites matières polymères pouvant être constituées d'une matière polymère unique ayant à la fois des propriétés de gonflement et de gélification : ladite matrice comportant éventuellement en outre divers adjuvants habituels destinés notamment à lui conférer de bonnes caractéristiques de compression.

De tels adjuvants sont notamment des diluants comme le lactose, des lubrifiants comme le stéarate de magnésium, des agents de granulation comme la polyvinylpyrrolidone, des agents améliorant les propriétés d'écoulement comme par exemple la silice colloïdale et des agents colorants comme par exemple l'oxyde de fer.

Ces adjuvants pourront être incorporés à la matrice précitée en une quantité comprise entre 25 % et 60 % en poids, rapportée au poids total de la matrice.

Une matière polymère ayant un pouvoir de gonflement élevé, susceptible d'être utilisée dans le cadre de la présente invention, est par exemple une carboxyméthylcellulose de sodium réticulée, une hydroxypropylcellulose réticulée, une hydroxyméthylpropylcellulose de poids moléculaire élevé, un polyméthylmétacrylate, une polyvinylpyrrolidone réticulée ou encore un alcool polyvinylique de poids moléculaire élevé.

Une matière polymère gélifiable susceptible d'être utilisée dans le cadre de la présente invention est par exemple la méthylcellulose, la carboxyméthylcellulose, une hydroxypropylméthylcellulose de faible poids moléculaire, un alcool polyvinylique de faible poids moléculaire, un polyoxyéthvlèneglycol ou encore une polyvinylpyrrolidone non réticulée.

Dans le cadre de la présente invention, on utilisera de préférence une matière polymère unique ayant à la fois des propriétés de gonflement et de gélification. Une telle matière est avantageusement une hydroxypropylméthylcellulose de poids moléculaire élevé, telle que le produit connu sous la dénomination commerciale METHOCEL® K100M, ce composé conférant en outre d'excellentes propriétés de viscosité au mélange final.

D'une façon générale, la matière polymère ayant un pouvoir de gonflement élevé et la matière polymère gélifiable représenteront ensemble environ 40 à 60 %, et de préférence 49.0 % en poids du poids total de la matrice précitée.

Le rapport pondéral entre la matière polymère ayant un pouvoir de gonflement élevé et la matière polymère gélifiable peut varier dans une large mesure.

Dans certains cas, pour obtenir les taux de dissolution in vitro recherchés, il peut être nécessaire d'incorporer à la matrice, une substance lipophile destinée à réguler la vitesse de libération de la molsidomine.

Une telle substance lipophile est avantageusement un composé lipidique hydrophobe comme les huiles de ricin hydrogénées (Cutina®), les alcools stéarilique, cétostéarilique, cétylique, des mono, di, triglycérides tel le palmitostéarate de glyceryl, le monoléate de glyceryl, la paraffine solide.

Dans le cadre de la présente invention, on utilisera de préférence un béhénate de glycérol tel que le produit connu sous la dénomination commerciale COMPRITOL® 888 ATO, ce composé permettant une excellente régulation de la perméabilité de la matrice.

La substance lipophile précitée peut être présente au sein de la matrice en une quantité de l'ordre de 12 % à 25 % en poids, rapportée au poids total de la matrice.

Le système de libération de la molsidomine utilisé dans le cadre de la présente invention peut être préparé par les procédés traditionnels bien connus de l'homme de métier comportant des étapes de mélange, de tamisage, de granulation, de séchage et de compression.

Pour obtenir le profil de libération souhaité, il peut être avantageux de donner à la matrice une forme géométrique assurant un allongement de la libération sur une durée de 24 heures.

Ainsi, le système de libération utilisé dans le cadre de la présente invention peut être constitué d'une matrice multicouche comprenant au moins une couche " active " incorporant la molsidomine, associée au moins à une couche " inactive " constituée de préférence essentiellement des mêmes matières que la couche active, mais n'incorporant pas de molsidomine.

Selon un mode de réalisation actuellement préféré, la forme galénique conforme à la présente invention est réalisée sous la forme d'un comprimé comprenant une couche active intercalée entre deux couches inactives.

L'invention sera illustrée plus en détail par les exemples suivants, donnés uniquement à titre illustratif.

### EXEMPLE 1

### Préparation d'une forme galénique conforme à l'invention sous forme de comprimés multicouches dosés à 16 mg

On a réalisé une forme galénique conforme à la présente invention se présentant sous la forme d'un comprimé comprenant une couche active intercalée entre deux couches inactives dont les dimensions sont les suivantes :
- diamètre du comprimé : 8,0 mm
- épaisseur de la couche active intercalaire : environ 2.1 mm
- épaisseur de chaque couche inactive : environ 1,55 et 1.95 mm.

Chacune de ces couches a été préparée en utilisant des matières essentiellement identiques, dans les quantités indiquées au Tableau I pour un comprimé.

**TABLEAU I**

| INGREDIENTS | COUCHE ACTIVE mg | COUCHES INACTIVES | |
|---|---|---|---|
| | | mg | mg |
| - MOLSIDOMINE | 16.00 | - | - |
| - METHOCEL K100M Premium | 60.00 | 39.88 | 31.90 |
| - COMPRITOL 888 ATO | 20.00 | 13.50 | 10.80 |
| - MANNITOL 60 | 5.00 | - | - |
| - PLASDONE K29-32 | 3.70 | 5.00 | 4.00 |
| - MAGNESIUM STEARATE | 1.06 | 1.00 | 0.80 |
| - AEROSIL 200 | 0,44 | 0.50 | 0.40 |
| - SICOVIT GELB 10 | - | 0.25 | 0.20 |
| - LACTOSE PUL VIS H₂O | - | 39.87 | 31.90 |
| MASSE TOTALE | 106.20 | 100.00 | 80.00 |

La couche active a été préparée de la façon suivante :

On a mélangé intimement la molsidomine, la matière polymère (METHOCEL® K100 M), la substance lipophile (COMPRITOL® 888 ATO), une charge hydrophile (MANNITOL® 60) et un agent de granulation (PLASDONE® K29-32) dans un mélangeur approprié.

Par ailleurs, on a préparé une solution d'éthanol à 95 % que l'on utilise pour humecter le mélange pulvérulent obtenu précédemment.

La masse homogène ainsi obtenue a été soumise à une granulation et un séchage en lit d'air fluidisé pour obtenir, après calibrage, un granulé.

Le granulé homogène ainsi obtenu a été mélangé à un agent améliorant l'écoulement (AEROSIL® 200) et à un agent lubrifiant (STEARATE DE MAGNESIUM) puis, comprimé.

Les couches inactives ont été réalisées en suivant un protocole identique à celui décrit précédemment pour la couche active, la pression lors des étapes de compression étant choisie pour obtenir un comprimé parfaitement homogène (pression d'environ 1000 kg/cm²).

### EXEMPLE 2

### Détermination du profil de dissolution in vitro d'une forme galénique conforme à l'invention

En utilisant la méthode décrite dans la Pharmacopée Européenne, 3ème Edition (ou U.S.P. XXIV), on a mesuré le taux de dissolution in vitro d'une forme galénique conforme à l'invention telle que celle préparée à l'exemple 1.

Les essais ont été réalisés dans les conditions expérimentales suivantes :
. Appareil Sotax AT7 équipé de pales
. Vitesse de rotation : 50 tpm
. Température du milieu de dissolution : 37°C
. Filtration: filtre Wathman GF-D
. Dosage : spectrophotométrie UV à environ 286 ou 311 nm
. Spectro : Hitachi U-3000 avec cellule en quartz de 1 cm
. Milieu de dissolution : 500 ml de HCl.0.1 N (pH acide).

Les résultats suivants ont ainsi été obtenus :
18 % de molsidomine libérée après 1 heure
27 % de molsidomine libérée après 2 heures
57 % de molsidomine libérée après 6 heures
88 % de moisidomine libérée après 12 heures
96 % de molsidomine libérée après 18 heures
100 % de molsidomine libérée après 24 heures.

### EXEMPLE 3

### Etude comparée des principales caractéristiques pharmacocinétiques de formulations à base de molsidomine

Afin de mettre en évidence les avantages et l'intérêt de la forme galénique conforme à la présente invention, par rapport aux formes galéniques de la molsidomine antérieurement connues, on a mesuré les principales caractéristiques pharmacocinétiques des trois formulations suivantes :
. Formulation à base de molsidomine dosée à 4 mg correspondant au produit actuellement commercialisé en Belgique sous la dénomination CORVATON® 4 mg.
. Formulation à base de molsidomine dosée à 8 mg actuellement commercialisée en Belgique sous la dénomination CORVATARD®.
. Formulation conforme à la présente invention dosée à 16 mg (réalisée conformément à l'exemple 1 ).

En utilisant des protocoles expérimentaux bien connus de l'homme de l'art, on a mesuré pour chacune de ces formulations les différents paramètres suivants:
- Cmax : concentration plasmatique maximale.
- Tmax : temps pour lequel le Cmax est observé.
- AUC 0-t : aire sous la courbe entre le temps 0 et le temps t
- T½ : temps de demi-vie d'élimination
- MRT : temps moyen de présence de la substance dans l'organisme.

Dans le cas de la formulation conforme à la présente invention, ces caractéristiques pharmacocinétiques ont été déterminées sur de jeunes volontaires sains à jeûn, puis après repas.

On a regroupé au Tableau II les résultats ainsi obtenus.

**TABLEAU II**

| | **MOLSIDOMINE** **4 mg** **(n=12)** | **MOLSIDOMINE** **8 mg** **(n=12)** | **MOLSIDOMINE** **16 mg (à jeûn)** **(n=10)** | **MOLSIDOMINE** **16 mg (repas)** **(n=10)** |
|---|---|---|---|---|
| **Cmax (ng/ml)** | 40.13 ± 19.03 | 3.80 ± 15.44 | 34.19 ± 25.37 | 34.76± 15.03 |
| **Tmax (h)** | 0.75 ± 0.34 | 1.67 ± 0.94 | 3.00 ±1.41 | 4.60 ± 2.10 |
| **AUC O-t (ng.h/ml)** | 103.6 ± 79.40 | 195.5 ± 124.5 | 372.5 ± 278.1 | 327.7 ± 166.9 |
| **AUCO-**^{**00**} **(ng.h/ml)** | 114.8 ± 89.40 | 229.8 ± 154.4 | 527.2 ± 466.6 | 409.3 ± 194.1 |
| **T ½ (h)** | 1.55 ± 0.50 | 3.35 ± 0.78 | 11.87 ± 10.35 | 11.54 ± 10.21 |
| **MRT (h)** | 2.64 ± 0.74 | 5.81 ± 1.47 | 1 8.99 ± 11.84 | 17.58 ± 11.33 |

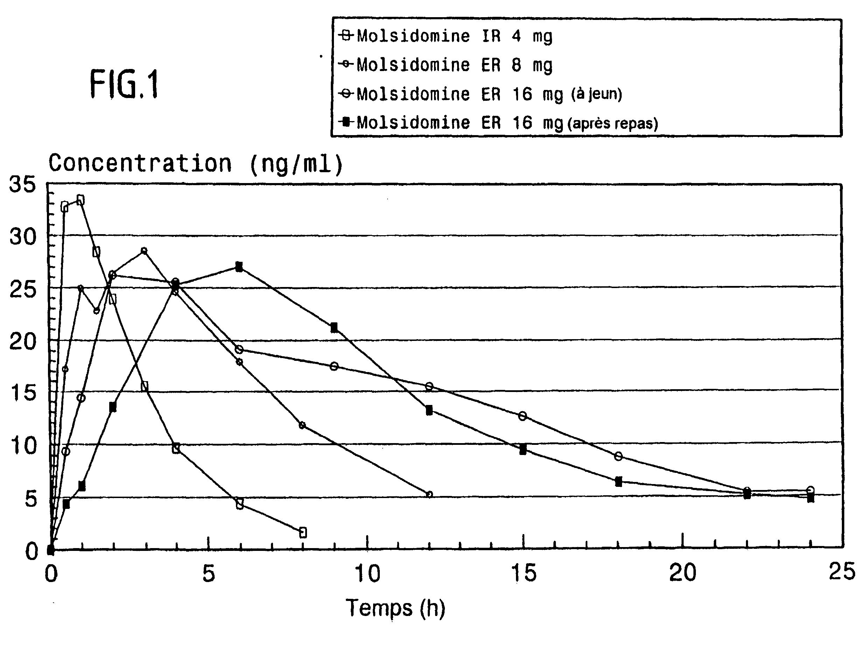

On a par ailleurs représenté à la figure 1, pour chacune des formulations étudiées. révolution de la concentration plasmatique en fonction du temps.

Les résultats obtenus montrent que la formulation dosée à 4 mg fournit une concentration plasmatique pendant environ 4 à 5 heures. la formulation dosée à 8 mg pendant environ 10 à 12 heures et la formulation selon l'invention dosée à 16 mg pendant environ 24 heures.

On peut constater que la concentration plasmatique maximale est relativement équivalente pour les trois formulations, et se situe entre 33 et 40 ng/ml.

Le résultat obtenu avec la formulation conforme à la présente invention est tout à fait inattendu puisque l'allongement de l'effet thérapeutique n'implique pas une diminution significative du Cmax. II n'existe pas de différence statistiquement significative entre la forme galénique selon la présente invention et les formes traditionnelles (ANOVA suivie de tests post-hoc de Bonferonni).

Ces résultats montrent en outre que la formulation conforme à l'invention garantit une efficacité comparable à celle des formulations connues, même pendant les premières heures post-administration avec une entrée rapide dans la zone thérapeutique endéans environ 30 min. (à jeûn) et 90 min. (après repas).

### EXEMPLE 4

### Etude comparée des corrélations entre la cinétique de libération in vitro dans différents milieux et la cinétique d'absorption in vivo

| Corrélation in vivo - in vitro | Molsidomine 4 mg | Molsidomine 8mg | Moisidomine 16 mg (à jeûn) | Molsidomine 16 mg (repas) |
|---|---|---|---|---|
| à pH 6.8 | 0.958 | 0.835 | 0.712 | 0.761 |
| à pH acide (milieu HCl 0. 1 n) | 0.877 | 0.855 | 0.748 | 0.812 |
| pH dépendance* | NA | 0.817 | 0.719 | 0.755 |

| | | | | |
|---|---|---|---|---|
| NA = non applicable * Note : les termes " pH dépendance " signifient que lors du test de dissolution vitro les comprimés soumis au test sont maintenus à pH 1.3 pendant 1 heure à pH 5,0 pendant 30 minutes à pH 6.3 pendant 3 heures à pH 7,0 pendant le reste du temps | | | | |

Tous les coefficients de corrélation sont significatifs (p < 0.01 ; Pearson's test). La meilleure corrélation (0.958) est obtenue avec la forme immédiate (molsidomine 4 mg) ce qui semble assez logique. En effet. il est connu que plus une forme galénique se complexifie et plus il est difficile de trouver une corrélation entre la libération in vitro et l'absorption in vivo. Pour la molsidomine 8 mg la corrélation reste très élevée (0.855). Pour la molsidomine 16 mg la corrélation est la meilleure (0.812) lorsque l'on prend en compte la cinétique après repas et la dissolution en milieu acide. De manière générale, pour les formes à libération prolongée, les corrélations sont toujours meilleures lorsque la libération est effectuée en milieu acide. Cela s'explique par le fait qu'une grosse partie de la cinétique de la molsidomine dépend de l'absorption stomacale.

### EXEMPLE 5

### Etude de la concentration plasmatique résultant de l'administration d'une formulation de molsidomine selon l'invention en fonction du temps chez des patients âgés angineux

Trente-trois patients coronariens présentant une angine de poitrine stable ont reçu une dose unique de molsidomine 16 mg (formulation selon l'exemple 1), prise à 8 heures du matin au petit déjeûner. L'effectif comportait 22 hommes et 11 femmes, l'âge moyen étant de 62,6 ± 1,3 ans (valeurs extrêmes : 49 à 73 ans). Répartis en 7 groupes, les patients ont subi une prise de sang pour dosage de molsidomine, respectivement 3, 6. 10, 14. 18, 22 et 24 heures après l'ingestion du médicament.

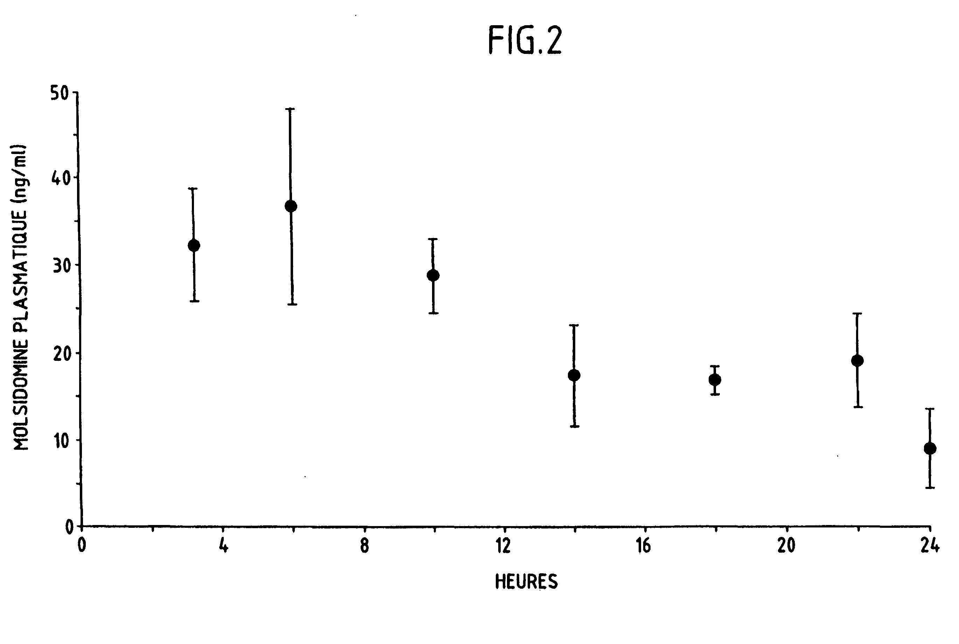

La figure 2 présente la concentration plasmatique moyenne de molsidomine et son erreur standard pour chacun des 7 groupes de patients étudiés.

Comme le montre cette figure, l'allure cinétique observée chez les patients âgés est très corrélée à celle observée chez les jeunes sujets volontaires sains et représentée à la figure 1. De plus, les valeurs de Cmax, Tmax et temps de demi-vie sont comparables.

On constate en outre que :
- la concentration moyenne maximale est de 36,0 ± 10,8 ng/ml :
- la concentration moyenne la plus élevée est observée dans le groupe 2, c'est-à-dire 6 heures après ingestion de la molsidomine ;
- la concentration décroît lentement, soit de 50 % en 8 heures ;
- la concentration plasmatique en molsidomine se maintient en plateau pendant 8 heures, de +14 à +22 heures après administration avec une concentration moyenne oscillant de 16,5 à 18,1 ng/ml :
- une concentration résiduelle de 8,5 ± 4.3 ng/ml est encore observée 24 heures après administration d'une dose unique de molsidomine 16 mg.

### EXEMPLE 6

### Etude de la corrélation entre efficacité clinique et concentration plasmatique d'une formulation de molsidomine selon l'invention.

Dix patients coronariens présentant une angine de poitrine stable ont subi un sevrage de tout traitement anti-angineux (dérivés nitrés à action prolongée, molsidomine, antagonistes calciques et/ou bêtabloquants). d'une durée minimale de 3 jours, plus longue en cas de prise de bêtabloquants ; seuls étaient autorisés pendant cette période, la prise de comprimés d'isosorbide-dinitrate 5 mg sublingual ou l'usage d'un spray de nitroglycérine.

Ces patients ont ensuite reçu une dose unique de molsidomine à 16 mg (formulation selon l'exemple 1) ou de placebo, selon une technique de randomisation en double-aveugle croisée comportant une période de sevrage d'au moins 2 jours complets.

L'effectif comportait 8 hommes et 2 femmes, l'âge moyen étant de 61,3 ± 3,1 ans (valeurs extrêmes : 49 à 73).

Répartis en 7 groupes, les patients ont subi une prise de sang pour dosage de molsidomine après une épreuve d'effort sur cyclo-ergomètre, respectivement 3, 6, 10, 14, 18, 22 et 24 heures après l'ingestion du médicament ou du placebo.

L'épreuve d'effort comportait une charge initiale de 30 Watts, avec accroissement de 30 Watts toutes les 3 minutes jusqu'à l'arrêt du test pour symptômes angineux, atteinte de la fréquence cardiaque maximale théorique, fatigue musculaire ou pour motif de sécurité (trouble du rythme ou de conduction intracardiaque, chute tensionnelle > 20 mm Hg, dépression du segment ST ≥ 3 mm) ; l'ECG et la pression artérielle étaient enregistrés au repos et tout au long du test d'effort.

L'efficacité clinique de la formulation selon l'invention a été quantifiée par :
a) la différence en durée totale d'exercice, exprimée en secondes, sous placebo et sous molsidomine.
b) la différence en travail total effectué, exprimé par la somme des produits Watts x min calculés pour chaque palier d'effort, sous placebo et sous molsidomine.

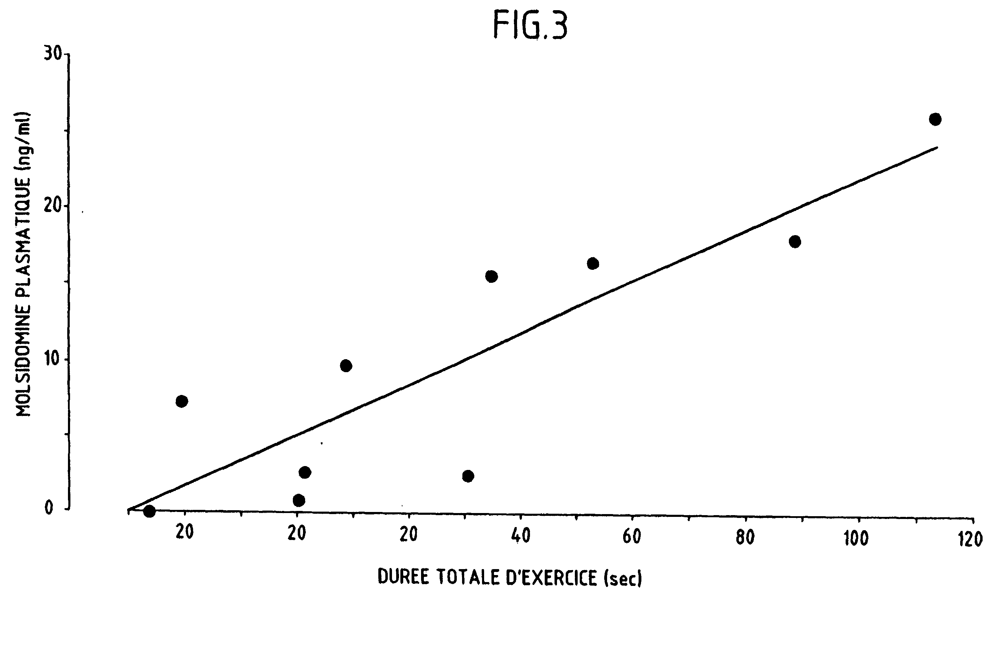

La figure 3 présente les concentrations plasmatiques de molsidomine observées chez chacun des 10 patients et les différences correspondantes en durée totale d'exercice.

L'équation de la droite de régression entre la variable X (différence entre la durée totale d'exercice sous placebo et sous molsidomine) et la variable Y (concentration plasmatique en molsidomine) est : Y = 0,18 X + 5,35

Le coefficient de corrélation de Pearson r est de 0,88 (P < 0,001 ), ce qui correspond à un coefficient de détermination r² de 0,77, 77 % de la variance de l'efficacité clinique pouvant être expliqués par la concentration plasmatique en molsidomine.

Un modèle quadratique ou cubique n'améliore pas le coefficient de corrélation.

Si l'on considère comme cliniquement significative une différence de 30 secondes entre épreuves d'effort effectuées sous placebo et molsidomine, il apparaît que la concentration plasmatique en molsidomine nécessaire pour ce niveau d'efficacité est de 10,75 ng/ml, taux encore pratiquement atteint 24 heures après une prise unique de molsidomine 16 mg. Selon l'invention (voir figure 2).

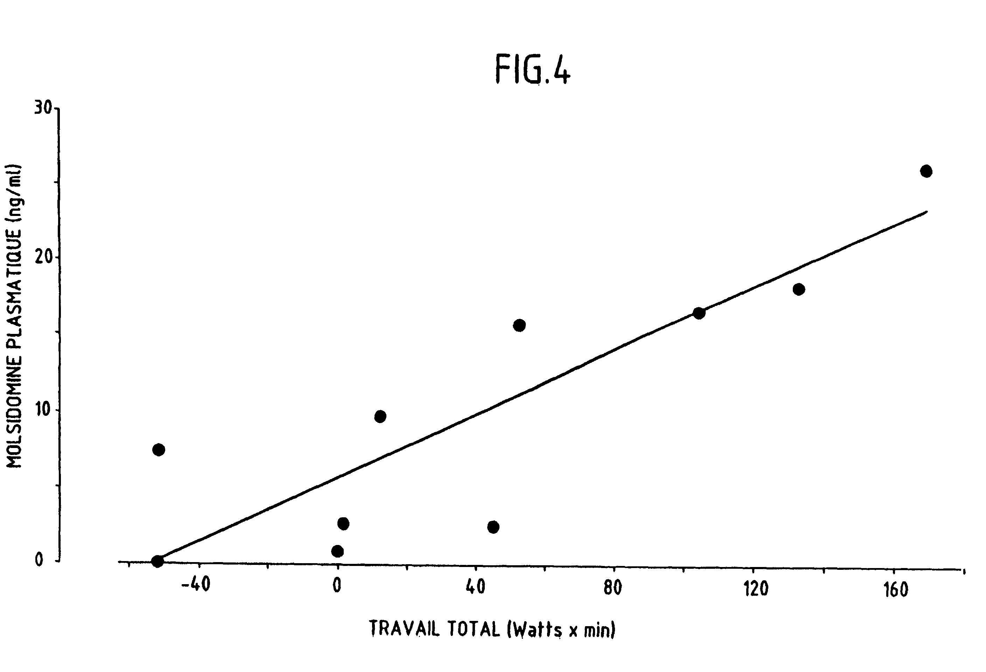

La figure 4 présente les concentrations plasmatiques de molsidomine observées chez chacun des 10 patients et les différences correspondantes en travail total effectué.

L'équation de la droite de régression entre la variable X (différence entre le travail total effectué sous placebo et sous molsidomine) et la variable Y (concentration plasmatique en molsidomine) est : Y = 0,11X + 5,90.

Le coefficient de corrélation de Pearson r est de 0,86 (p = 0,002), ce qui correspond à un coefficient de détermination r² de 0,74) 74 % de la variance de l'efficacité clinique pouvant être expliqués par la concentration plasmatique en molsidomine.

Un modèle quadratique ou cubique n'améliore pas le coefficient de corrélation.

Si l'on considère comme cliniquement significative une différence de 50 Watts x min entre épreuves d'effort effectuées sous placebo et molsidomine, il apparaît que la concentration plasmatique en molsidomine nécessaire pour ce niveau d'efficacité est de 11,40 ng/ml, taux encore pratiquement atteint 24 heures après une prise unique de molsidomine 16 mg selon l'invention (voir figure 2).

### EXEMPLE 7

### Etude de l'efficacité clinique d'une formulation de molsidomine selon l'invention

Deux cent vingt deux patients présentant un angor d'effort stable ont participé à une étude plurinationale multicentrique, randomisée en double aveugle et contrôlée par placebo.

Après administration d'une dose unique de molsidomine à 16 mg. (formulation selon l'exemple 1) ou de placebo, ils ont subi une épreuve d'effort sur cyclo-ergomètre dans un délai variant de 2 h à 24 h après la prise du médicament. La capacité d'effort (travail total effectué, exprimé en Watts x min) est significativement plus élevée sous molsidomine que sous placebo, tant statistiquement (p < 0,001) que cliniquement (amélioration moyenne de 53 Watts x min).

L'administration d'une même dose unique journalière de molsidomine 16 mg (formulation selon l'exemple 1) pendant 2 semaines, entraîne une amélioration de performance physique comparable à celle observée après administration aiguë, significative tant statistiquement (p < 0.001) que cliniquement (amélioration moyenne de 58 Watts x min).

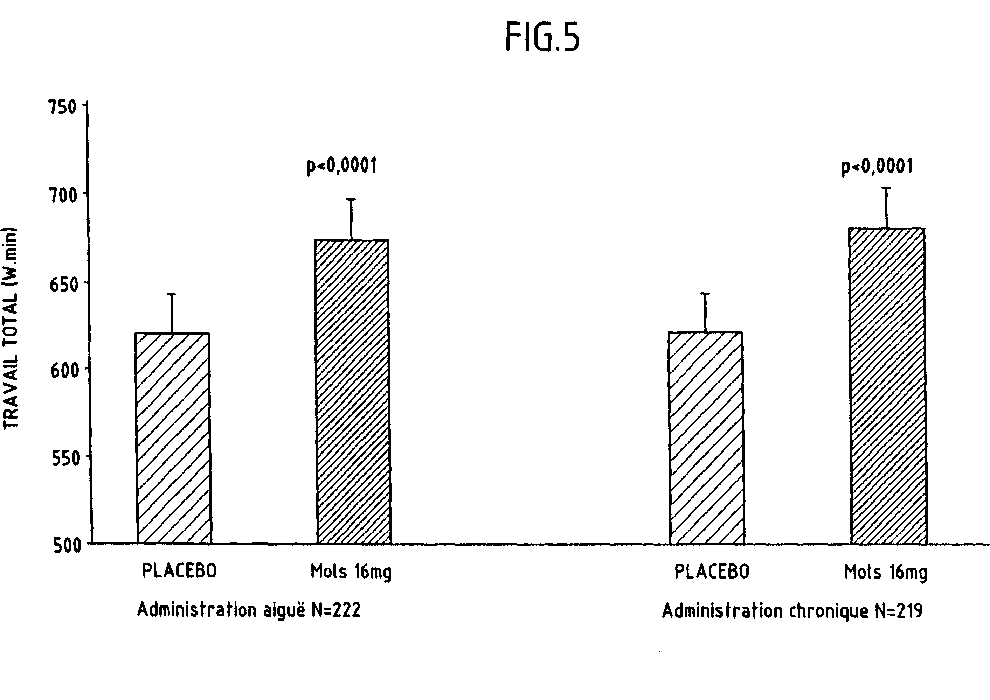

Ces résultats attestent de l'absence d'accoutumance à la molsidomine 16 mg après traitement prolongé (Figure 5). Ils indiquent également une efficacité thérapeutique s'étendant sur 24 heures.

On notera que les améliorations cliniques observées dans cette étude sont en parfaite concordance avec les concentrations plasmatiques en molsidomine cliniquement efficaces déduites de la corrélation travail total / molsidomine plasmatique présentée à la Figure 4.

Les résultats obtenus aux exemples 5, 6 et 7 qui précèdent. démontrent l'originalité de la nouvelle forme galénique de molsidomine selon l'invention.

Les principaux avantages de cette forme, par comparaison aux formes existantes peuvent être résumés de la façon suivante :
- Maintien d'un plateau de concentration plasmatique en molsidomine élevé (16,5 à 18,1 ng/ml) pendant 8 heures (de + 14 heures à + 22 heures après l'ingestion du médicament).
- Plateau de concentration plasmatique assurant une efficacité clinique significative chez des patients coronariens avec angine de poitrine stable, comme le démontre l'étroite corrélation entre augmentation de la capacité d'effort et concentration sanguine en molsidomine, amélioration qui se maintient 24 heures après l'administration du médicament.
- Absence d'accoutumance à la molsidomine, une efficacité clinique significative persistant après 2 semaines de traitement de patients coronariens avec angine de poitrine stable.

## Revendications

1. Forme galénique orale solide à libération prolongée de la molsidomine, **caractérisée en ce qu'**elle contient une quantité thérapeutiquement efficace de molsidomine ou de l'un de ses métabolites actifs choisi parmi les composés SIN-1 et SIN-1A et **en ce qu'**elle présente un taux de dissolution in vitro [mesuré spectrophotométriquement à 286 ou 311 nm selon la méthode décrite dans la Pharmacopée Européenne, 3ème édition (ou U.S.P. XXIV) à 50 t.p.m. dans 500 ml d'un milieu HCl 0,1 N, à 37°C) de :
- 15 à 25 % de molsidomine libérée après 1 heure
- 20 à 35 % de molsidomine libérée après 2 heures
- 50 à 65 % de molsidomine libérée après 6 heures
- 75 à 95 % de molsidomine libérée après 12 heures
- > 85 % de molsidomine libérée après 18 heures
- > 90 % de molsidomine libérée après 24 heures,
le pic plasmatique de molsidomine obtenu in vivo se présentant dans les 2,5 à 5 heures, de préférence dans les 3 à 4 heures, suivant l'administration orale de ladite forme et ayant une valeur comprise entre 25 et 40 ng/ml de plasma.

2. Forme galénique selon la revendication 1, **caractérisée en ce qu'**elle comprend de 14 à 24 mg, et de préférence de 16 à 20 mg de molsidomine par unité de dosage.

3. Forme galénique selon la revendication 1 ou 2, **caractérisée en ce qu'**elle se présente sous forme de comprimés, sous forme pluriparticulaire ou sous forme de sphéroïdes.

4. Forme galénique selon l'une des revendications 1 à 3, **caractérisée en ce qu'**elle comprend un système de libération de la molsidomine constitué d'une matrice à libération prolongée, ou bien encore d'une formulation traditionnelle comprenant un enrobage permettant la libération prolongée de la molsidomine.

5. Forme galénique selon la revendication 4, **caractérisée en ce que** le système de libération précité est constitué d'une matrice active comprenant, mélangées à la molsidomine ou à l'un de ses métabolites actifs, une matière polymère ayant un pouvoir de gonflement élevé au contact de l'eau ou de liquides aqueux, une matière polymère gélifiable, lesdites matières polymères pouvant être constituées d'une matière polymère unique ayant à la fois des propriétés de gonflement et de gélification ; ladite matrice comportant éventuellement en outre divers adjuvants habituels destinés notamment à lui conférer de bonnes caractéristiques de compression.

6. Forme galénique selon la revendication 5, **caractérisée en ce que** les adjuvants précités sont choisis parmi des diluants comme le lactose, des lubrifiants comme le stéarate de magnésium, des agents de granulation comme la poiyvinylpyrrolidone, des agents améliorant les propriétés d'écoulement comme par exemple la silice colloïdale et des agents colorants comme par exemple l'oxyde de fer.

7. Forme galénique selon l'une des revendications 5 ou 6, **caractérisée en ce que** la matrice active précitée est constituée d'hydroxypropylméthylcellulose de poids moléculaire élevé, ayant à la fois des propriétés de gonflement et de gélification, telle que notamment le produit connu sous la dénomination commerciale METHOCEL® K100M.

8. Forme galénique selon l'une des revendications 5 à 7, **caractérisée en ce que** la matrice active précitée contient en outre une substance lipophile contribuant à réguler la vitesse de libération de la molsidomine.

9. Forme galénique selon la revendication 8, **caractérisée en ce que** la substance lipophile précitée est choisie parmi les composés lipidiques hydrophobes comme les huiles de ricin hydrogénées (Cutina), les alcools stéarilique, cétostéarilique, cétylique, des mono, di, triglycérides tel le palmitostéarate de glyceryl, le monoléate de glyceryl, la paraffine solide.

10. Forme galénique selon la revendication 9, **caractérisée en ce que** la substance lipophile précitée est un bénénate de glycérol, tel que le produit connu sous la dénomination commerciale COMPRITOL® 888 ATO.

11. Forme galénique selon l'une des revendications précédentes, **caractérisée en ce qu'**elle se présente sous la forme de comprimés multicouches comprenant de préférence une couche active incorporant la molsidomine, associée à au moins une couche inactive constituée de préférence essentiellement des mêmes matières que la couche active mais n'incorporant pas de molsidomine.

12. Forme galénique selon la revendication 11, **caractérisée en ce qu'**elle comporte une couche active intercalée entre deux couches inactives.

## Patentansprüche

1. Oral anzuwendende feste galenische Form mit verzögerter Freisetzung des Molsidomins, **dadurch gekennzeichnet, dass** sie eine therapeutisch wirksame Menge von Molsidomin oder einem seiner wirksamen Metaboliten enthält, welche aus den Zusammensetzungen SIN-1 und SIN-1A gewählt sind, und dadurch, dass sie einen Lösungsgehalt in vitro [spektrophotometrisch gemessen bei 286 oder 311 nm gemäß dem Verfahren, welches in Pharmacopée Européenne, 3. Auflage (oder U.S.P. XXIV) beschrieben ist, bei 50 U/min in 500 ml eines Mediums HCl 0,1 N bei 37°C] aufweist von:
- 15 bis 25 % freigesetztem Molsidomin nach 1 Stunde
- 20 bis 35 % freigesetztem Molsidomin nach 2 Stunden
- 50 bis 65 % freigesetztem Molsidomin nach 6 Stunden
- 75 bis 95 % freigesetztem Molsidomin nach 12 Stunden
- > 85 % freigesetztem Molsidomin nach 18 Stunden
- > 90 % freigesetztem Molsidomin nach 24 Stunden,
wobei der plasmatische Peak des in vivo erhaltenen Molsidomins innerhalb von 2,5 bis 5 Stunden auftritt, vorzugsweise innerhalb 3 bis 4 Stunden, gemäß der oralen Anwendung der Form, und einen Wert zwischen 25 und 40 ng/ml des Plasmas aufweist.

2. Galenische Form gemäß Anspruch 1, **dadurch gekennzeichnet, dass** sie 14 bis 24 mg, und vorzugsweise 16 bis 20 mg Molsidomin pro Dosiereinheit aufweist.

3. Galenische Form gemäß Anspruch 1 oder 2, **dadurch gekennzeichnet, dass** sie in Form von Tabletten, in multipartikulärer Form oder in Form von Sphäroiden vorliegt.

4. Galenische Form gemäß einem der Ansprüche 1 bis 3, **dadurch gekennzeichnet, dass** sie ein System zur Freisetzung des Molsidomins aufweist, welches aus einer Matrix zur verzögerten Freisetzung besteht, oder auch aus einer herkömmlichen Formulierung, die eine Umhüllung aufweist, welche die verzögerte Freisetzung des Molsidomins ermöglicht.

5. Galenische Form gemäß Anspruch 4, **dadurch gekennzeichnet, dass** das System zur Freisetzung aus einer aktiven Matrix gebildet wird, welche, mit dem Molsidomin oder einem seiner wirksamen Metaboliten vermischt, ein Polymermaterial aufweist, das ein erhöhtes Quellvermögen aufweist bei Kontakt mit Wasser oder wässrigen Flüssigkeiten, ein gelierfähiges Polymermaterial, wobei die Polymermaterialien aus einem einzigen Polymermaterial gebildet sein können, welches gleichzeitig Eigenschaften des Quellens und des Gelierens aufweist; wobei die Matrix gegebenenfalls des weiteren verschiedene übliche Zusatzstoffe aufweist, die insbesondere dazu bestimmt sind, ihr gute Kompressionseigenschaften zu verleihen.

6. Galenische Form gemäß Anspruch 5, **dadurch gekennzeichnet, dass** die Zusatzstoffe aus Verdünnungsmitteln wie Lactose, Gleitmitteln wie Magnesiumstearat, Granuliermitteln wie Polyvinylpyrrolidon, Mitteln zur Verbesserung der Fließeigenschaften, wie beispielsweise kolloidale Kieselerde, und Färbemitteln, wie beispielsweise Eisenoxid, gewählt werden.

7. Galenische Form gemäß einem der Ansprüche 5 oder 6, **dadurch gekennzeichnet, dass** die aktive Matrix aus Hydroxypropylmethylcellulose mit erhöhtem Molekulargewicht gebildet wird, welche gleichzeitig Eigenschaften des Quellens und des Gelierens aufweist, wie insbesondere das unter der Verkaufsbezeichnung bekannte Produkt ME-THOCEL®K100M.

8. Galenische Form gemäß einem der Ansprüche 5 bis 7, **dadurch gekennzeichnet, dass** die aktive Matrix des weiteren eine lipophile Substanz enthält, welche dazu beiträgt, die Geschwindigkeit der Freisetzung des Molsidomins zu regulieren.

9. Galenische Form gemäß Anspruch 8, **dadurch gekennzeichnet, dass** die lipophile Substanz aus den hydrophoben lipiden Verbindungen gewählt ist, wie hydrierten Rizinuslölen (Cutina), Stearyl-, Cetostearyl-, Cetylalkoholen, Mono-, Di-, Triglyceriden wie Glycerin-Palmitostearat, Glycerin-Monoleat, festem Paraffin.

10. Galenische Form gemäß Anspruch 9, **dadurch gekennzeichnet, dass** die lipophile Substanz ein Glycerin-Behenat ist, wie das unter dem Markennamen bekannte Produkt COMPRITOL®888 ATO.

11. Galenische Form gemäß einem der vorausgegangenen Ansprüche, **dadurch gekennzeichnet, dass** diese in Form von Mehrschicht-Tabletten vorliegt, welche vorzugsweise eine aktive Schicht aufweisen, die das Molsidomin enthält, verbunden mit wenigstens einer inaktiven Schicht, die vorzugsweise im wesentlichen aus den gleichen Materialien gebildet ist, wie die aktive Schicht, aber kein Molsidomin enthält.

12. Galenische Form gemäß Anspruch 11, **dadurch gekennzeichnet, dass** diese eine aktive Schicht aufweist, die zwischen zwei inaktiven Schichten eingefügt ist.

## Claims

1. Sustained-release solid oral galenical form of molsidomine, **characterized in that** it contains a therapeutically effective amount of molsidomine or one of its active metabolites chosen among compounds SIN- 1 and SIN-1A, and **in that** it has an in vitro dissolution rate [measured spectrophotometrically at 286 or 311 nm by the method described in the European Pharmacopoeia, 3rd edition (or USP XXIV), at 50 rpm, in 500 ml of a 0.1 N HCl medium, at 37°C] of:
- 15 to 25% of molsidomine released after 1 hour
- 20 to 35% of molsidomine released after 2 hours
- 50 to 65% of molsidomine released after 6 hours
- 75 to 95% of molsidomine released after 12 hours
- >85% of molsidomine released after 18 hours
- >90% of molsidomine released after 24 hours,
the plasma peak of molsidomine obtained in vivo occurring 2.5 to 5 hours, preferably 3 to 4 hours, following the administration of said form, and having a value of between 25 and 40 ng/ml of plasma.

2. Galenical form according to claim 1, **characterized in that** it comprises from 14 to 24 mg, preferably from 16 to 20 mg, of molsidomine per dosage unit.

3. Galenical form according to claim 1 or 2, **characterized in that** it is in the form of tablets, in a multiparticulate form or in the form of spheroids.

4. Galenical form according to one of claims 1 to 3, **characterized in that** it comprises a molsidomine release system consisting of a sustained-release matrix or of a traditional formulation comprising a coating allowing sustained release of the molsidomine.

5. Galenical form according to claim 4, **characterized in that** the aforementioned release system consists of an active matrix comprising, mixed with the molsidomine or one of its active metabolites, a polymeric material with a high swelling capacity in contact with water or aqueous liquids, and a gellable polymeric material, it being possible for said polymeric materials to consist of a single polymeric material having both swelling and gelling properties, said matrix optionally also containing various customary adjuvants, especially for imparting good compression characteristics thereto.

6. Galenical form according to claim 5, **characterized in that** the aforementioned adjuvants are selected from diluents such as lactose, lubricants such as magnesium stearate, granulating agents such as polyvinylpyrrolidone, flow improvers such as colloidal silica, and colorants such as iron oxide.

7. Galenical form according to claim 5 or 6, **characterized in that** the aforementioned active matrix consists of high molecular hydroxypropylmethylcellulose having both swelling and gelling properties, such as, in particular, the product known under the trade name METHOCEL® K100M.

8. Galenical form according to one of claims 5 to 7, **characterized in that** the aforementioned active matrix also contains a lipophilic substance in order to help regulate the rate of release of the molsidomine.

9. Galenical form according to claim 8, **characterized in that** the aforementioned lipophilic substance is selected from hydrophobic lipid compounds such as hydrogenated castor oils (Cutina), stearyl, cetostearyl and cetyl alcohols, mono-, di- and triglycerides such as glyceryl palmitostearate and glyceryl monooleate, and solid paraffin.

10. Galenical form according to claim 9, **characterized in that** the aforementioned lipophilic substance is a glycerol behenate such as the product known under the trade name COMPRITOL® 888 ATO.

11. Galenical form according to one of the preceding claims, **characterized in that** it is in the form of multilayer tablets preferably comprising an active layer incorporating the molsidomine, associated with at least one inactive layer preferably consisting essentially of the same materials as the active layer, but not incorporating molsidomine.

12. Galenical form according to claim 11, **characterized in that** it comprises one active layer intercalated between two inactive layers.
